# EUROPÄISCHE PATENTSCHRIFT

(11) **EP 1 926 450 B1**
(45) Veröffentlichungstag und Bekanntmachung des Hinweises auf die Patenterteilung: **16.12.2009**
(21) Anmeldenummer: 07820923.6
(22) Anmeldetag: 04.10.2007
(51) Int. Cl.: A61C 13/083, A61K 6/06, C04B 41/52, C04B 41/89

(54) **VERFAHREN ZUR HERSTELLUNG EINES KERAMISCHEN FORMTEILS**
METHOD FOR THE PRODUCTION OF A CERAMIC MOLDED PART
PROCEDE DE FABRICATION D'UNE PIECE MOULEE EN CERAMIQUE

(30) Priorität: 05.10.2006 EP 06020897
(43) Veröffentlichungstag der Anmeldung: 04.06.2008
(73) Patentinhaber: Vita Zahnfabrik H. Rauter GmbH & Co. KG, 79713 Bad Säckingen (DE)
(72) Erfinder: STEPHAN, Marc, 79539 Lörrach (DE); THIEL, Norbert, 79713 Bad Säckingen (DE); BOJEMÜLLER, Enno, 79713 Bad Säckingen (DE)
(74) Vertreter: Meyers, Hans-Wilhelm
(86) Internationale Anmeldenummer: PCT/EP2007/060546
(87) Internationale Veröffentlichungsnummer: WO 2008/040780

(56) Entgegenhaltungen:
- WO-A-02/47616
- WO-A-2005/103339
- US-A- 2 463 551
- US-A1- 2002 152 768

## Beschreibung

Gegenstand der vorliegenden Erfindung ist ein Verfahren zur Herstellung eines keramischen Formteils aus einer Suspension/Dispersion mit einem Feststoffanteil und einem fluiden Anteil sowie ein poröser, selbsttragender Träger, der zur Herstellung eines keramischen Formteils verwendet werden kann.

Im Bereich der Herstellung von Zahnersatz hat sich in den letzten Jahren in die auf Sadoun zurückgehende Schlickertechnik für hochwertigen Zahnersatz bewährt. Die Methode ist im Einzelnen in der EP-A-0 241 384 im Detail beschrieben. Diese Technik stellt zwar hohe Anforderungen an den Techniker, ist aber erlernbar und führt dann zu ausgezeichneten Ergebnissen.

US-A-2,463,551 offenbart ein Verfahren zur Herstellung von künstlichen Zähnen und Teilen davon. Die dort offenbarte Methode ist zur Herstellung von Kunststoffzähnen, also keinen dentalkeramischen Restaurationen, geeignet.

US-A-4,155,964 offenbart eine Technik zum Aufbau dentalkeramischer Formteile. Dabei wird ein repulverisiertes Dentalporzellan in einer Aufschlämmung durch Eintauchen der Form in eine Aufschlämmung des Dentalporzellans, das auch einen Binder enthält, mit dem Material beaufschlagt und dann in einer Ausführungsform durch Absaugen der Flüssigkeit der Aufschlämmung unterstützend getrocknet. Eine andere Ausführungsform wird dadurch verifiziert, dass in einem Behälter ein poröses Abformmaterial angeordnet ist und die Negativform der abzuformenden Masse in dieses Material eingedrückt wird und die entstandene Mulde mit der Aufschlämmung gefüllt wird und dann entweder durch Zentrifugieren oder Absaugen der Feststoffe in der Aufschlämmung an der inneren Peripherie der eingedrückten Form abgeschieden wird. Nachteilig an der letztgenannten Variation ist, dass es aufwendig ist, das hergestellte Formteil aus der Mulde wieder zu entfernen und weiteren Verarbeitungsschritten zuzuführen. Das Abdruckmaterial ist ein relativ hartes, poröses Formmaterial, welches nicht selbsttragend ist, da es in einem Behälter angeordnet ist.

Es besteht ein Bedürfnis an einem Verfahren, das den notwendigen Schlickeraufbauunabhängiger von der Geschicklichkeit eines Zahntechnikers gestaltet.

Gelöst wird das der Erfindung zu Grunde liegende Problem durch ein Verfahren zur Herstellung eines dental Keramischen Formteils aus einer Suspension/Dispersion mit einem Feststoffanteil und einem fluiden Anteil durch Absetzen von Feststoffanteil an der Peripherie eines porösen, selbsttragenden Trägers, der mindestens teilweise in die Suspension/Dispersion eintaucht und die Verkleinerung der Form des herzustellenden Formkörpers besitzt, wobei
- der Feststoffanteil oxidkeramische Partikel enthält,
- der poröse, selbsttragende Träger in einer nicht in die Suspension/Dispersion eintauchenden Zone mit einer Abführung lösbar verbunden ist,
- die Suspension/Dispersion mittels einer positiven Druckdifferenz aus einem in der Suspension/Dispersion herrschenden Druck und einem in der Abführung herrschenden Druck in Richtung des porösen, selbsttragenden Trägers bewegt wird, wobei
unter Absetzen von Feststoffanteilen an der Peripherie des porösen, selbsttragenden Trägers der fluide Anteil des Suspension/Dispersion in den porösen, selbsttragenden Träger eindringt.

Das erfindungsgemäße Verfahren ermöglicht eine schnelle Abfolge in der Herstellung mehrerer verschiedener Formkörper, wobei auf die herkömmliche Schlickertechnik mittels Auftragen durch Pinsel verzichtet werden kann. Die Auftragetechnik des Schlickers mit Pinsel ist langwierig und bedarf einiger Übung. Gemäß der Erfindung kann die Schichtdicke der Formteile vorteilhafterweise durch die Wahl der Dauer des Unterdrucks reproduzierbar eingestellt werden. Gegenüber der konventionellen Schlickertechnik, bei der der Anwender Erfahrung im Umgang mit dem keramischen Schlicker benötigt, sind Fehler durch ungleichmäßiges Auftragen weitestgehend ausgeschlossen. Auch die Gefahr eines zu langen Trocknens einzelner, von Hand aufgetragener Schlickerschichten mit resultierender Bildung eines inhomogenen Gefüges (sog. Zwiebelschalen-Effekt), sowie das Entstehen von Poren wird verringert. Ein weiterer Vorteil des erfindungsgemäßen Verfahrens beruht auf der hervorragenden Passgenauigkeit der mit dem Schlicker hergestellten Gerüste. Die Schlickertechnik wird insbesondere in der In-Ceram^{®}-Technologie der Vita Zahnfabrik Henry Rauter GmbH & Co. KG eingesetzt und ist in Fachkreisen auch durch die hervorragenden Standzeiten der so hergestellten dentalen Restaurationen bekannt.

Der im erfindungsgemäßen Verfahren einsetzbare poröse, selbsttragende Träger besteht beispielsweise aus Gips, abgebundenen oder anderen in der Zahntechnik bekannten Stumpfmassen, insbesondere phosphatgebundenen Pulvermischungen, Quarz und/oder Metalloxiden.

Der selbsttragende, poröse Träger gemäß der Erfindung wird beispielsweise durch Eingießen von mit destilliertem Wasser angemischtem Gips oder anderer abbindbarer Materialien in einem Silikonabdruck des Meistermodells hergestellt. Nach dem Abbinden des abbindbaren Materials, im Fall des In-Ceram-Spezialgipses nach dem Erreichen der erforderlichen Abbindeexpansion, wird die nicht der Suspension zugewandten Seite, beispielsweise mit einem Gipstrimmer plan geschliffen, es kann aber auch, wie oben ausgeführt, eine Abführung angebracht werden. Möglich ist auch die Herstellung des selbsttragenden porösen Trägers durch Schleifen aus einem Block des betreffenden abbindbaren Materials auf Basis von CAD/CAM-Daten analog zum Schleifen einer Restauration aus keramischem Blockmaterial. Mit dieser Herstellmethode ist die Einstellung beliebiger Vergrößerungsfaktoren des Stumpfes realisierbar. Eine andere Möglichkeit zur Herstellung des selbsttragenden, porösen Stumpfes gemäß der Erfindung ist die Erzeugung eines vergrößerten Abdrucks des Meistermodells mit Hilfe eines geeigneten Expansionsmaterials zum Ausgleich des Sinterschrumpfes im Falle dichtzusinternder Materialien. Dieser Abdruck wird mit Gips oder ähnlichen Materialien ausgegossen, um einen vergrößerten selbsttragenden, porösen Stumpf zu erhalten.

Es kann vorteilhaft sein, dass der im erfindungsgemäßen Verfahren einsetzbare poröse, selbsttragende Träger mindestens eine Zone aufweist, die ein Eindringen des fluiden Anteils der Suspension/Dispersion verhindert.

In einer Ausführungsform des erfindungsgemäßen Verfahrens kann der poröse, selbsttragende Träger an einer nicht der Suspension/Dispersion zugewandten Seite eine im Wesentlichen planare Fläche aufweisen. Die im Wesentlichen planare Fläche kann dabei zweckmäßiger Weise mit der Abführung verbunden sein. Es kann vorteilhaft sein, dass die im Wesentlichen planare Fläche mit einem Flansch versehen ist.

In einer anderen Ausführungsform ist die im Wesentlichen planare Fläche mit einem Verbindungselement versehen. Als Verbindungselement kommen alle Elemente in Betracht mit deren Hilfe sich zwei getrennte Elemente lösbar verbinden lassen. Grundsätzlich kommen neben mechanischen Verbindungselementen auch chemische Verbindungselemente in Betracht. Der bereits erwähnte Flansch ist beispielsweise ein typisch mechanisches Verbindungselement, wohingegen als chemisches Verbindungselement beispielsweise eine Klebung in Betracht kommt.

In einer weiteren Ausführungsform weist der poröse, selbsttragende Träger mindestens eine Bohrung, insbesondere ein Sackloch, auf.

Die im erfindungsgemäßen Verfahren einzusetzende Suspension/Dispersion ist für den Bereich der dentalkeramischen Restauration typischerweise eine oxidkeramische Suspension/Dispersion. Die oxidkeramische Suspension/Dispersion kann dabei Aluminiumoxid, Zirkonoxid, Magnesium-Aluminiumoxid (Spinell), Yttriumoxid, Ceroxid, Silikate, Zirkonsilikat oder Kombinationen davon enthalten.

Die gemäß dem erfindungsgemäßen Verfahren anzuwendende positive Druckdifferenz kann beispielsweise durch Überdruck auf der Seite des porösen, selbsttragenden Trägers, die der Suspension/Dispersion zugewandt ist und/oder durch Unterdruck auf der Seite des porösen, selbsttragenden Trägers, die der Suspension/Dispersion abgewandt ist, bewirkt werden. Im Falle eines anzuwendenden Unterdrucks wird dieser insbesondere im Bereich der Abführung angelegt. Der Unterdruck kann zusätzlich oder auch nur im Bereich der Bohrung anliegen. Die Druckdifferenz beträgt zweckmäßiger Weise 100 bis 1000 mbar. Falls es notwendig erscheint kann die Druckdifferenz auch von dem genannten Bereich nach oben oder unten abweichen.

Es kann auch zur Abscheidung von sehr dicken Schichten eine größere Druckdifferenz nötig sein. Auch bei geringerer Porengröße des Stumpfmaterials müsste entsprechend ein stärkerer Unterdruck oder Überdruck angelegt werden. Insgesamt lässt sich der Materialtransport durch die Druckdifferenz steuern. Die für die einzelnen Zwecke geeigneten Druckdifferenzen kann der Fachmann in einfacher Weise durch Reihenversuche bestimmen.

Üblicherweise wird ein Unterdruck kleiner 500 mbar angelegt.

Die Porosität des porösen, selbsttragenden Trägers kann grundsätzlich durch Poren gebildet werden, die nicht größer als 20 Mikrometer. Typischerweise liegt die Größe der Poren in einem Bereich von 1 nm bis 10 µm (gemessen mittels Quecksilberporosimetrie).

Dem Fachmann ist es wohlbekannt, auch von den hier genannten Porengrößen abzuweichen. Z.B. kommen Materialien mit kleinerer Porengröße prinzipiell auch für die erfindungsgemäße Abscheidung in Frage. Sollten Materialien mit größeren Poren verwendet werden, könnte hier jedoch vorteilhafterweise eine Schicht aus einem Material mit geringerer Porengröße aufgebracht werden, um ein Eindringen der Keramikpartikel aus der Suspension oder Dispersion in das poröse, selbsttragende Stumpfmaterial zu verhindern.

In einer weiteren Ausführungsform der Erfindung kann nach Absetzen von Feststoffanteil an der Peripherie in einer Schicht mit einer vorbestimmten Schichtdicke der poröse, selbsttragende Träger in eine zweite Suspension/Dispersion eingetaucht werden, so dass die vorher gebildete Schicht mindestens teilweise in die zweite Suspension/Dispersion eintaucht und die beschriebenen Verfahrensschritte wiederholt werden. Selbstverständlich ist es möglich, gegebenenfalls weitere Eintauchvorgänge zu wiederholen, um mehrere Schichten aufzubauen.

In einer Ausführungsform des erfindungsgemäßen Verfahrens kann nach dem Aufbau einer oder mehrerer Schichten der poröse, selbsttragende Träger entnommen werden, wobei eine Druckdifferenz aufrechterhalten wird, um eine weitgehende Vortrocknung zu erzielen.

Es steht im Belieben des Fachmannes, dem erhaltenen keramischen Formkörper durch Nachbehandlung eine höhere Festigkeit zu verleihen. Erfindungsgemäß kommen dabei als Nachbehandlung ein Sinterbrand des keramischen Formkörper und/oder eine Infiltration mit anorganischen oder organischen Stoffen in Betracht.

Der Sinterbrand wird insbesondere bei Temperaturen von 600° C bis 1600° C durchgeführt. Als organische Stoffe zur Infiltration kommen insbesondere polymerisierbare organische Verbindungen in Betracht. Typischerweise werden Verbindungen verwendet, die mindestens eine ethylenisch ungesättigte Bindung besitzen. Hier bieten sich insbesondere Urethandimethacrylat (UDMA) und/oder Triethylenglykoldimethacrylat (TEGDMA) an.

Zu den anorganischen Stoffen gehören Infiltrationsgläser, insbesondere Lanthanboratgläser. Solche Infiltrationsgläser werden in der seit langem bekannten Schlickertechnik eingesetzt. Auch hier wird ausdrücklich auf die Offenbarung in der EP-A-0 241 384 Bezug genommen.

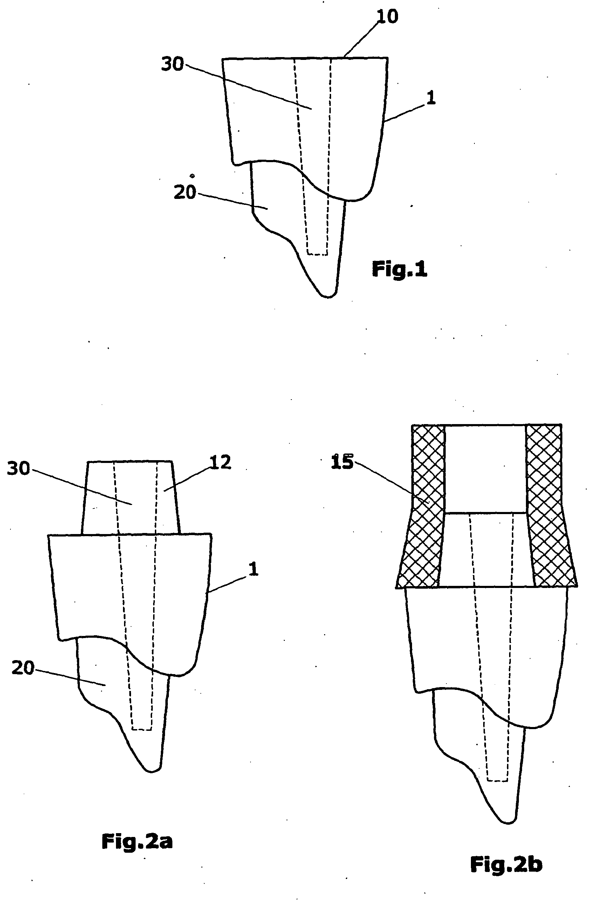

Das erfindungsgemäße Verfahren wird anhand der Figuren 1 bis 4 näher erläutert.

Gegenstand der vorliegenden Erfindung ist auch ein poröser, selbsttragender Träger 1 mit einer Basis 10 und einem Aufbau 20, der eine mit einem Feststoff zu belegende Form aufweist, und einer Bohrung 30, die durch die Basis 10 und den Aufbau 20 verläuft und als Sackloch ausgestaltet ist (Figur 1). An der Basis 10 ist, wie Figur 2a zeigt, eine Einrichtung 12 zum Anschließen einer Abführungseinrichtung 15 angeordnet. Die Abführungseinrichtung kann an eine Pumpe zur Erzeugung eines Unterdrucks angeschlossen sein (Figur 2b). Die Abführungseinrichtung kann beispielsweise ein Druckschlauch sein.

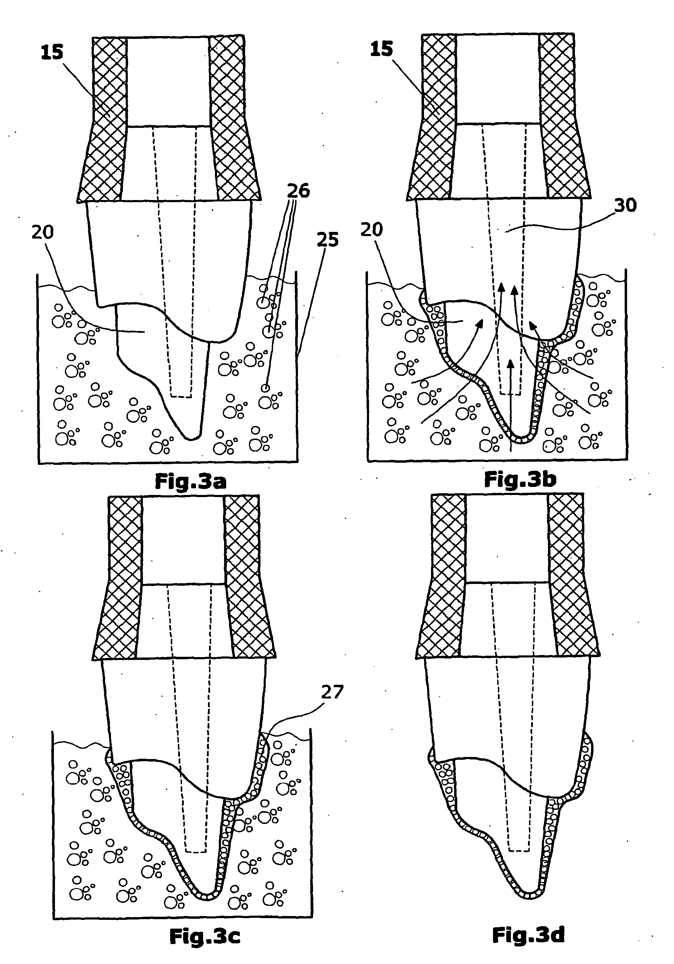

Die Figuren 3a bis 3d zeigen schematisiert den Ablauf des erfindungsgemäßen Verfahrens. In 3a wird die in Figur 2b gezeigte Vorrichtung in einen Behälter 25 getaucht, der die Suspension/Dispersion enthält, die zum Aufbau des Formkörpers eingesetzt wird. Die Teilchen 26 der Suspension/Dispersion werden nach Anlegen eines Unterdrucks an den erfindungsgemäßen porösen, selbsttragenden Träger angesaugt und scheiden sich an der Peripherie des Aufbaus 20 ab. Die Pfeile stellen dabei die Richtung des Druckgradienten hin zu niedrigeren Drücken dar. Nach Erreichen der gewünschten Schichtdicke an Schlickermaterial 27 an der Peripherie des Aufbaus und des porösen, selbsttragenden Trägers wird der Unterdruck abgestellt und die Anordnung aus dem Behälter entfernt. Die in Figur 3d gezeigte Anordnung kann dann nach Entfernung der Abführungseinrichtung in an sich bekannter Weise weiterverarbeitet werden. Durch Eintauchen des porösen, selbsttragenden Trägers in die Suspension/Dispersion kann die Höhe des Schlickeraufbaus gesteuert werden.

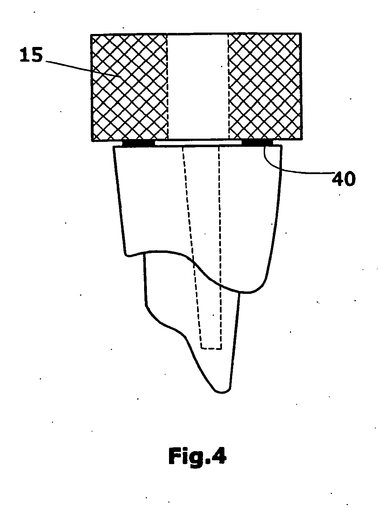

Figur 4 zeigt eine weitere Ausführungsform des Aufbaus, der in dem erfindungsgemäßen Verfahren eingesetzt werden kann.

Der selbsttragende poröse Träger 1 kann mit einem Befestigungsmaterial 40 an der Abführungseinrichtung 15 befestigt werden. Dies kann insbesondere mit folgenden Mitteln in einfacher Weise erfolgen:
- Doppelseitiges Klebeband in Ringform (wie z.B. Lochverstärkerringe), gegebenenfalls in einer Materialdicke, die Unebenheiten des Untergrunds ausgleicht
- Eine klebende, knetbare Masse, die in Ringform um die Bohrung 30 angelegt wird und durch manuelles Andrücken an der Abführungseinrichtung 15 befestigt wird.
- Geeignet ist auch ein magnetisches Klebeband, das mit einer magnetischen Abführungseinrichtung 15 verbunden wird
- oder die Verwendung einer wiederverwendbaren magnetischen Platte, die mit Hilfe einer der ersten beiden Beispiele an dem porösen Träger 1 befestigt wird und dann an die magnetische Abführungseinrichtung 15 angehängt wird.

Nach dem Abscheidevorgang soll sich der Gipsstumpf mit dem Befestigungsmaterial vorzugsweise rückstandsfrei von der Abführungseinrichtung 15 entfernen lassen, damit der nächste Gipsstumpf zur Abscheidung angehängt werden kann. Dies genannten Ausführungsformen sind Alternativen zur Befestigung des porösen selbsttragenden Trägers 1 mittels des Silikonschlauches an der Abführungseinrichtung 15.

Der erfindungsgemäße poröse, selbsttragende Träger eignet sich in hervorragender Weise zum Einsatz im erfindungsgemäßen Verfahren.

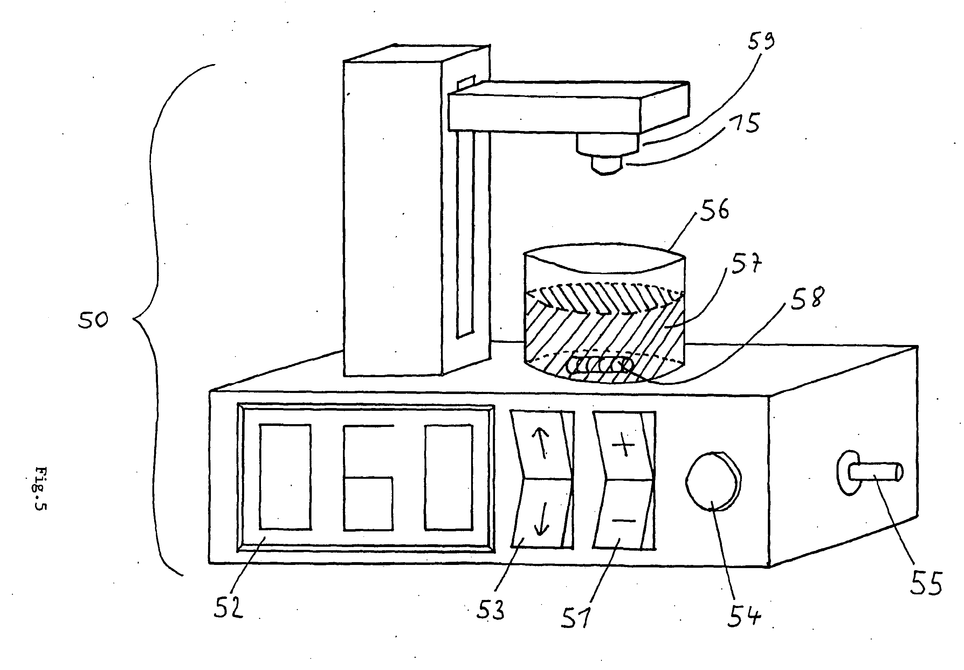

Die Figur 5 zeigt im einzelnen eine Vorrichtung 50 gemäß einer besonderen Ausführungsform der Erfindung.
51: Kippschalter zum Einstellen der Saugzeit
52: Anzeige der Saugzeit
53: Kippschalter zum vertikalen Auf- und Abbewegen des Halters
54: Start-Knopf zum Einschalten des Vorganges (Vakuum startet, nach Ablauf der vorgegebenen Zeit schaltet sich das Vak. automatisch ab und der Halter wird hochgefahren.)
55: Vakuum- oder Druckluft Anschluss
56: Schlickerbehälter
57: Schlicker (Suspension/Dispersion)
58: Magnetrührfisch
15: Abführungseinrichtung
59: vertikal bewegliche Halterung mit Abführungseinrichtung

Die Erfindung anhand des folgenden Beispiels näher erläutert.

Das erfindungsgemäße Verfahren basiert auf der Schlickerabscheidung auf der Oberfläche eines mit einer Bohrung versehenen porösen Gipsstumpfes. Der Gipsstumpf ist an der Unterseite plan geschliffen und so dimensioniert, dass ein flexibler Schlauch als Adapter, insbesondere Silikonschlauch, vakuumdicht darübergezogen (oder damit verbunden) werden kann. Der mit dem flexiblen Schlauch versehene Gipsstumpf wird auf einen Flansch geschoben oder mit einem Adapter verbunden, der an einer beweglichen Halterung zur Aufnahme des Gipsstumpfs befestigt ist. Die Halterung ist vorzugsweise motorisch bewegbar und kann in den darunter angeordneten Behälter, der den Schlicker enthält, eingefahren werden. Der Schlicker wird mittels eines Rührers, beispielsweise eines Magnetrührmotors mit einem Magnetrührfisch, in Bewegung gehalten, um die Sedimentation des Schlickers zu verhindern. An dem Halter ist eine Vakuumleitung angeordnet, die mit einer Vorrichtung zur Erzeugung von Unterdruck, z.B. einer Pumpe verbunden ist. Danach wird der Gipsstumpf manuell oder durch automatische Steuerung in den Schlicker eingefahren bis die Präparationsgrenze vollständig bedeckt ist. Nach dem Einfahren des Gipsstumpfs in den Schlicker wird Unterdruck angelegt. Dabei wird während des Abscheidevorgangs der Schlicker nicht gerührt. Nach der vorgegebenen Zeitspanne wird Druckausgleich geschaffen, und zwar während sich der Gipsstumpf mit dem abgeschiedenen Material noch im Schlicker befindet. Danach kann die Halterung bis zur Ausgangsstellung wieder hochgefahren werden. Der Gipsstumpf mit den darauf abgeschiedenen Käppchen kann vom Flansch oder Adapter abgenommen und weiter bearbeitet werden. Danach steht das Gerät zur weiteren Verarbeitung zur Verfügung.

Typischerweise kann durch Variation der Unterdruckzeit die Schichtdicke des Schlickers variiert werden. So kann beispielsweise bei Erzeugung einer 0,7 mm starken Frontzahnkappe eine Saugzeit von ca. 20 s bis 60 s, insbesondere ca. 40 s, zur Erzeugung einer gleichstarken Seitenzahnkappe eine Saugzeit 30 bis 90 s, insbesondere ca. 60 s eingehalten werden.

## Patentansprüche

1. Verfahren zur Herstellung eines dentalkeramischen Formteils aus einer Suspension/Dispersion mit einem Feststoffanteil und einem fluiden Anteil durch Absetzen von Feststoffanteil an der Peripherie eines porösen, selbsttragenden Trägers, der mindestens teilweise in die Suspension/Dispersion eintaucht und die Verkleinerung der Form des herzustellenden Formkörpers besitzt, wobei
- der Feststoffanteil oxidkeramische Partikel enthält,
- der poröse, selbsttragende Träger in einer nicht in die Suspension/Dispersion eintauchenden Zone mit einer Abführung lösbar verbunden ist,
- die Suspension/Dispersion mittels einer positiven Druckdifferenz aus einem in der Suspension/Dispersion herrschenden Druck und einem in der Abführung herrschenden Druck in Richtung des porösen, selbsttragenden Trägers bewegt wird, wobei
unter Absetzen von Feststoffanteil an der Peripherie des porösen, selbsttragenden Trägers der fluide Anteil der Suspension/Dispersion in den porösen, selbsttragenden Träger eindringt.

2. Verfahren nach Anspruch 1, wobei der poröse, selbsttragende Träger aus abgebundenem Gips, Stumpfmassen, insbesondere phosphatgebundene Pulvermischungen, Quarz, Metalloxiden besteht.

3. Verfahren nach Ansprüch 1 und/oder 2, wobei der poröse, selbsttragende Träger mindestens eine Zone aufweist, die ein Eindringen des fluiden Anteils der Suspension/Dispersion verhindert.

4. Verfahren nach mindestens einem der Ansprüche 1 bis 3, wobei der poröse, selbsttragende Träger an einer nicht der Suspension/Dispersion zugewandten Seite im Wesentlichen eine planare Fläche aufweist.

5. Verfahren nach Anspruch 4, wobei die im Wesentlichen planare Fläche mit der Abführung verbunden ist.

6. Verfahren nach Anspruch 5, wobei die im Wesentlichen planare Fläche mit einem Flansch versehen ist.

7. Verfahren nach Anspruch 5, wobei die im Wesentlichen planare Fläche mit einem Verbindungselement versehen ist.

8. Verfahren nach Anspruch 7, wobei das Verbindungselement durch eine Klebung gebildet wird.

9. Verfahren nach mindestens einem der Ansprüche 1 bis 8, wobei der poröse, selbsttragende Träger mindestens eine Bohrung aufweist.

10. Verfahren nach Anspruch 9, wobei die Bohrung ein Sackloch ist.

11. Verfahren nach mindestens einem der Ansprüche 1 bis 8, wobei die Suspension/Dispersion eine oxidkeramische Suspension/Dispersion ist.

12. Verfahren nach Anspruch 9, wobei die oxidkeramische Suspension/Dispersion Aluminiumoxid, Zirkonoxid, Magnesium-Aluminiumoxid (Spinell), Yttriumoxid, Ceroxid, Silikate, Zirkonsilikat oder Kombinationen davon enthält.

13. Verfahren nach mindestens einem der Ansprüche 1 bis 10, wobei die positive Druckdifferenz durch Überdruck auf der Seite des porösen, selbsttragenden Trägers, die der Suspension/Dispersion zugewandt ist und/oder durch Unterdruck auf der Seite des porösen, selbsttragenden Trägers, die der Suspension/Dispersion abgewandt ist, bewirkt wird.

14. Verfahren nach Anspruch 13, wobei der Unterdruck im Bereich der Abführung angelegt wird.

15. Verfahren nach Anspruch 14, wobei der Unterdruck auch im Bereich der Bohrung anliegt.

16. Verfahren nach mindestens einem der Ansprüche 13 bis 15, wobei die Druckdifferenz 100 bis 1000 mbar ist.

17. Verfahren nach mindestens einem der Ansprüche 13 bis 15, wobei ein Unterruck kleiner 500 mbar angelegt ist.

18. Verfahren nach mindestens einem der Ansprüche 1 bis 17, wobei die Porosität des porösen, selbsttragenden Trägers 1 nm bis 10 µm gemessen mittels Quecksilberporosimetrie beträgt.

19. Verfahren nach mindestens einem der Ansprüche 1 bis 18, wobei nach Absetzen von Feststoffanteil an der Peripherie in einer Schicht mit einer vorbestimmten Schichtdicke der poröse, selbsttragende Träger in eine zweite Suspension/Dispersion eingetaucht wird, so dass die vorher gebildete Schicht mindestens teilweise in die zweite Suspension/Dispersion eintaucht und die Verfahrensschritte von Anspruch 1 wiederholt werden und gegebenenfalls weitere Eintauchvorgänge wiederholt werden zum Aufbau mehrerer Schichten.

20. Verfahren nach mindestens einem der Ansprüche 1 bis 19, wobei nach der Entnahme des porösen, selbsttragenden Trägers mit einer oder mehreren Schichten die Druckdifferenz aufrecht erhalten wird, um eine weitgehende Vortrocknung zu erzielen.

21. Verfahren nach mindestens einem der Ansprüche 1 bis 20, wobei der erhaltene keramische Formkörper durch Nachbehandlung eine höhere Festigkeit erhält.

22. Verfahren nach Anspruch 19, wobei die Nachbehandlung ein Sinterbrand des keramischen Formkörpers ist und/oder durch eine Infiltration mit anorganischen oder organischen Stoffen ist.

23. Verfahren nach Anspruch 20, wobei der Sinterbrand bei Temperaturen von 600° C bis 1600° C durchgeführt wird.

24. Verfahren nach Anspruch 21, wobei die organischen Stoffe zur Infiltration polymerisierbare organische Verbindungen, insbsondere Urethandimethacrylat (UDMA) und Triethylenglykoldimethacrylat (TEGDMA) und die anorganischen Stoffe Infiltrationsgläser, insbesondere Lanthanboratgläser sind.

25. Poröser, selbsttragender Träger (1) zur Herstellung eines dentalkeramischen Formteils, mit einer Basis (10) und einem Aufbau (20), der eine mit einem Feststoff zu belegende Form aufweist, und einer Bohrung (30), die durch die Basis (10) und den Aufbau (20) verläuft und als Sackloch ausgestaltet ist, wobei im Bereich der Basis (10) eine Abführungseinrichtung (15) zur Abführung von fluiden Anteilen einer aus Feststoffanteil und fluiden Anteilen bestehenden Suspension/Dispersion vorgesehen ist.

## Claims

1. A process for the preparation of a dental ceramic molded part from a suspension/dispersion with a solids content and a fluid content by depositing solids content at the periphery of a porous self-supporting support that is at least partially immersed in the suspension/dispersion and has the same shape as the molded part to be prepared, but with a reduced size, wherein:
- the solids content contains oxide-ceramic particles;
- the porous self-supporting support is detachably connected with a discharge in a zone that is not immersed in the suspension/dispersion;
- the suspension/dispersion is moved towards the porous self-supporting support by means of a positive pressure difference between a pressure prevailing in the suspension/dispersion and a pressure prevailing in the discharge;
wherein the fluid content of the suspension/dispersion enters in the porous self-supporting support with depositing solids content at the periphery of the porous self-supporting support.

2. The process according to claim 1, wherein said porous self-supporting support consists of cured gypsum, stump compositions, especially phosphate-bound powder mixtures, quartz, metal oxides.

3. The process according to claim 1 and/or 2, wherein said porous self-supporting support has at least one zone that prevents the fluid content of the suspension/dispersion from entering.

4. The process according to at least one of claims 1 to 3, wherein said porous self-supporting support has a generally planar surface on a side not facing towards the suspension/dispersion.

5. The process according to claim 4, wherein said generally planar surface is connected with said discharge.

6. The process according to claim 5, wherein said generally planar surface is provided with a flange.

7. The process according to claim 5, wherein said generally planar surface is provided with a connecting element.

8. The process according to claim 7, wherein said connecting element is represented by adhesive bonding.

9. The process according to at least one of claims 1 to 8, wherein said porous self-supporting support has at least one bore.

10. The process according to claim 9, wherein said bore is a blind bore.

11. The process according to at least one of claims 1 to 8, wherein said suspension/dispersion is an oxide-ceramic suspension/dispersion.

12. The process according to claim 9, wherein said oxide-ceramic suspension/dispersion contains alumina, zirconia, magnesium aluminum oxide (spinel), yttria, ceria, silicates, zirconium silicate or combinations thereof.

13. The process according to at least one of claims 1 to 10, wherein said positive pressure difference is caused by an overpressure on the side of the porous self-supporting support facing towards the suspension/dispersion, and/or by a reduced pressure on the side of the porous self-supporting support facing away from the suspension/dispersion.

14. The process according to claim 13, wherein said reduced pressure is applied in the region of the discharge.

15. The process according to claim 14, wherein said reduced pressure is also applied in the region of the bore.

16. The process according to at least one of claims 13 to 15, wherein said pressure difference is from 100 to 1000 mbar.

17. The process according to at least one of claims 13 to 15, wherein a reduced pressure of less than 500 mbar is applied.

18. The process according to at least one of claims 1 to 17, wherein the porosity of the porous self-supporting support is from 1 nm to 10 µm as measured by means of mercury porosimetry.

19. The process according to at least one of claims 1 to 18, wherein after depositing a solids content on the periphery in a layer having a predetermined layer thickness, the porous self-supporting support is immersed in a second suspension/dispersion, so that the previously formed layer is at least partially immersed in the second suspension/dispersion, and the process steps of claim 1 are repeated, and optionally further immersion processes are repeated to build up several layers.

20. The process according to at least one of claims 1 to 19, wherein the pressure difference is maintained after the porous self-supporting support with one or more layers is withdrawn, to achieve extensive predrying.

21. The process according to at least one of claims 1 to 20, wherein the ceramic molded part obtained is provided with a higher strength by an aftertreatment.

22. The process according to claim 19, wherein said aftertreatment comprises the sintering of the ceramic molded part and/or infiltration with inorganic or organic materials.

23. The process according to claim 20, wherein said sintering is performed at temperatures of from 600 °C to 1600 °C.

24. The process according to claim 21, wherein said organic materials for infiltration are polymerizable organic compounds, especially urethane dimethacrylate (UDMA) and triethylene glycol dimethacrylate (TEGDMA), and said inorganic materials are infiltration glasses, especially lanthanum borate glasses.

25. A porous self-supporting support (1) for the preparation of a dental ceramic molded part with a base (10) and a superstructure (20), which has a shape to be coated with a solid, and a bore (30) which runs through the base (10) and the superstructure (20) and is designed as a blind bore, wherein a discharge means (15) for discharging fluid contents of a suspension/dispersion consisting of a solids content and a fluid content is provided in the region of the base (10).

## Revendications

1. Procédé de fabrication d'une pièce moulée de céramique dentaire à partir d'une suspension/dispersion contenant une fraction de matières solides et une fraction de matières fluides consistant à laisser se déposer la fraction de matières solides sur la périphérie d'un support autoporteur poreux, lequel support est immergé au moins en partie dans la suspension/dispersion et représente, en taille réduite, la forme de la pièce moulée à fabriquer, dans lequel :
- la fraction de matières solides contient des particules de céramique oxydée,
- le support autoporteur poreux, au niveau d'une zone non immergée dans la suspension/dispersion, est relié de manière amovible à un dispositif d'aspiration,
- la suspension/dispersion est déplacée dans la direction du support autoporteur poreux au moyen d'une différence de pression positive entre une pression établie dans la suspension/dispersion et une pression établie dans le dispositif d'aspiration,
procédé dans lequel :
la fraction fluide de la suspension/dispersion pénètre dans le support autoporteur poreux tout en laissant la fraction de matières solides se déposer au niveau de la périphérie du support autoporteur poreux.

2. Procédé selon la revendication 1, dans lequel le support autoporteur poreux est composé de plâtre pris, de masses pour prothèses dentaires, en particulier de mélanges de poudres à liant phosphate, de quartz, d'oxydes métalliques.

3. Procédé selon la revendication 1 et/ou 2, dans lequel le support autoporteur poreux présente au moins une zone qui empêche une pénétration de la fraction fluide de la suspension/dispersion.

4. Procédé selon au moins l'une quelconque des revendications 1 à 3, dans lequel le support autoporteur poreux présente une surface essentiellement planaire située au niveau d'un côté opposé à la suspension/dispersion.

5. Procédé selon la revendication 4, dans lequel la surface essentiellement planaire est reliée au dispositif d'aspiration.

6. Procédé selon la revendication 5, dans lequel la surface essentiellement planaire est reliée à une bride.

7. Procédé selon la revendication 5, dans lequel la surface essentiellement planaire est pourvue d'un élément de liaison.

8. Procédé selon la revendication 7, dans lequel l'élément de liaison est réalisé par un collage.

9. Procédé selon au moins l'une quelconque des revendications 1 à 8, dans lequel le support autoporteur poreux présente au moins un alésage.

10. Procédé selon la revendication 9, dans lequel l'alésage est un trou borgne.

11. Procédé selon au moins l'une quelconque des revendications 1 à 8, dans lequel la suspension/dispersion est une suspension/dispersion de particules de céramique oxydée.

12. Procédé selon la revendication 9, dans lequel la suspension/dispersion de particules de céramique oxydée contient de l'oxyde d'aluminium, de l'oxyde de zirconium, de l'oxyde de magnésium-oxyde d'aluminium (spinelle), de l'oxyde d'yttrium, de l'oxyde de cérium, des silicates, du silicate de zirconium ou des combinaisons de ceux-ci.

13. Procédé selon au moins l'une quelconque des revendications 1 à 10, dans lequel la différence de pression positive est obtenue au moyen d'une surpression sur le côté du support autoporteur poreux orienté vers la suspension/dispersion et/ou au moyen d'un vide partiel sur le côté du support autoporteur poreux opposé à la suspension/dispersion.

14. Procédé selon la revendication 13, dans lequel le vide partiel est appliqué dans la zone du dispositif d'aspiration.

15. Procédé selon la revendication 14, dans lequel le vide partiel est également appliqué dans la zone de l'alésage.

16. Procédé selon au moins l'une quelconque des revendications 13 à 15, dans lequel la différence de pression va de 100 à 1000 mbar.

17. Procédé selon au moins l'une quelconque des revendications 13 à 15, dans lequel on applique un vide partiel inférieur à 500 mbar.

18. Procédé selon au moins l'une quelconque des revendications 1 à 17, dans lequel la porosité du support autoporteur poreux mesurée au moyen d'une porosimétrie au mercure va de 1 nm à 10 µm.

19. Procédé selon au moins l'une quelconque des revendications 1 à 18, dans lequel, une fois que la fraction de particules solides s'est déposée sur la périphérie selon une couche ayant une épaisseur prédéterminée, le support autoporteur poreux est immergé dans une deuxième suspension/dispersion de sorte que la couche formée auparavant est immergée, au moins en partie, dans la deuxième suspension/dispersion et les étapes de procédé selon la revendication 1 sont répétées et, si nécessaire, d'autres processus d'immersion sont réalisés de manière répétée afin de constituer plusieurs couches.

20. Procédé selon au moins l'une quelconque des revendications 1 à 19, dans lequel la différence de pression est maintenue afin d'obtenir un pré-séchage étendu après le retrait du support autoporteur poreux comportant une ou plusieurs couches.

21. Procédé selon au moins l'une quelconque des revendications 1 à 20, dans lequel la pièce moulée de céramique est soumise à un traitement afin d'obtenir une solidité supérieure.

22. Procédé selon la revendication 19, dans lequel le traitement ultérieur consiste en un frittage de la pièce moulée de céramique et/ou en une infiltration avec des matières inorganiques ou organiques.

23. Procédé selon la revendication 20, dans lequel le frittage est réalisé à des températures allant de 600°C à 1600°C.

24. Procédé selon la revendication 21, dans lequel les matières organiques destinées à l'infiltration sont des composés organiques polymérisables, en particulier le diméthacrylate d'uréthanne (UDMA) et le diméthacrylate de triéthylèneglycol (TEGDMA) et les matières inorganiques sont des verres d'infiltration, en particulier des verres de borate de lanthane.

25. Support autoporteur poreux (1) pour la fabrication d'une pièce moulée de céramique dentaire présentant une base (10) et une structure (20), lequel support présente une forme à revêtir d'une matière solide, ainsi qu'un alésage (30) s'étendant dans la base (10) et dans la structure (20) et réalisé sous forme de trou borgne, dans lequel un dispositif d'aspiration (15) est prévu dans la zone de la base (10) pour évacuer les fractions fluides d'une suspension/dispersion composée d'une fraction de matières solides et d'une fraction de matières fluides.
